# EUROPEAN PATENT SPECIFICATION

(11) **EP 4 402 694 B1**
(45) Date of publication and mention of the grant of the patent: **22.07.2026**
(21) Application number: 22868385.0
(22) Date of filing: 13.09.2022
(51) Int. Cl.: G16H 30/40, G06V 10/22, G06T 7/00, G06T 7/10

(54) **IDENTIFYING OBJECTS IN MAGNETIC RESONANCE IMAGES**
IDENTIFIZIERUNG VON OBJEKTEN IN MAGNETRESONANZBILDERN
IDENTIFICATION D'OBJETS DANS DES IMAGES À RÉSONANCE MAGNÉTIQUE

(30) Priority: 13.09.2021 US 202163243410 P
(43) Date of publication of application: 24.07.2024
(73) Proprietor: Board of Regents, The University of Texas System, Austin, TX 78701 (US)
(72) Inventor: SANDERS, Jeremiah W., Houston, Texas 77030 (US); FRANK, Steven J., Houston, Texas 77030 (US)
(74) Representative: Forresters IP LLP
(86) International application number: PCT/US2022/076333
(87) International publication number: WO 2023/039591

(56) References cited:
- US-A1- 2009 129 671
- US-A1- 2012 089 008
- US-A1- 2015 117 736
- US-A1- 2015 348 277
- US-A1- 2017 344 856
- US-B2- 7 672 498
- US-B2- 8 150 121
- ALLMAN DEREK ET AL: "Photoacoustic Source Detection and Reflection Artifact Removal Enabled by Deep Learning", vol. 37, no. 6, 1 June 2018 (2018-06-01), USA, pages 1464 - 1477, XP093050717, ISSN: 0278-0062, Retrieved from the Internet <URL:https://ieeexplore.ieee.org/ielaam/42/8370207/8345287-aam.pdf> DOI: 10.1109/TMI.2018.2829662

## Description

### CROSS-REFERENCE TO RELATED APPLICATION

This application claims priority to and the benefit of U.S. Provisional Application No. 63/243,410, filed September 13, 2021.

### FIELD

The present disclosure relates to analyzing magnetic resonance (MR) images. In particular, the disclosure involves analyzing MR images to detect radioactive seeds or organs.

### BACKGROUND

Prostate cancer is the second most common cancer in men and the second-leading cause of cancer death in men. Brachytherapy, in which radioactive material is surgically implanted into the body, is an effective treatment option for prostate cancer. Imaging is an important component of the prostate brachytherapy workflow. For low-dose-rate (LDR) prostate brachytherapy, imaging is used to perform radiation treatment planning and post-implant quality assessment. Computed tomography (CT) is the current standard imaging modality used for post-implant imaging. However, the similar electron density of the prostate and its surrounding organs limits soft tissue contrast in CT. This lack of contrast makes it difficult to delineate anatomic boundaries of interest for treatment planning and post-implant assessment. Moreover, photon starvation caused by significant absorption of x-rays passing through the radioactive seeds produces streak artifacts, which further complicates the ability to delineate the prostate and its surrounding organs at risk in post-implant CT.

In contrast to CT, magnetic resonance imaging (MRI) is well known for its high soft tissue contrast. Soft tissue contrast in MRI is governed by the pulse sequence selected for imaging, and the pulse sequence parameters can be tuned to adjust the contrast for a given imaging application. Such imaging has been used in MRI-assisted radiosurgery (MARS).

Images acquired for both radiation treatment planning and post-implant assessment may be used for radiation dosimetry purposes. Computing radiation dosimetry for MARS LDR prostate brachytherapy involves knowledge of both organ boundaries and radioactive seed locations. However, the task of automatic segmentation of the prostate and surrounding organs at risk under MRI in both pre-implant and post-implant MRI remains an active area of research. Improvements to MRI automatic segmentation of the prostate, surrounding organs, other organs (i.e., not limited to the prostate and surrounding organs), and localization of radioactive seeds are described herein.
US7672498 discloses a method is for correcting inhomogeneities in an image that is recorded from an examination object.

### SUMMARY

The invention is defined by the appended claims. Analysis of MR image for identifying objects such as organs and radioactive seeds is improved through image pre-processing and post-processing techniques. With pre-processing, MR image data can be analyzed to identify a set of biased voxels. These biased voxels can be removed and replaced with voxels having a random distribution centered around a specified value. The replacement voxels can approximate pseudo-noise and reduce bias that may affect the image analysis.

The post-processing techniques can involve handling the results of multiple machine learning models. Each voxel can be evaluated by multiple machine learning models to determine respective probabilities for whether the voxel is within a particular object (e.g., an organ or a seed). A probability of the voxel as corresponding to a particular object can be determined based on the respective probabilities of all machine learning models. The particular object may be displayed based on the determined probability.

Aspects described herein provide a method for identifying an object in an MR image of a subject. The method includes receiving an MR image that includes a set of image units. An image unit may be a pixel or a voxel. Each image unit includes one or more image unit values pertaining to a region of the subject. The image unit values form a first distribution. The method also includes determining a variation measure of the image unit values in the first distribution. The method further includes determining a centroid measure of the image unit values in the first distribution. Additionally, the method includes identifying a first set of image units having an image unit value that exceeds a threshold corresponding to the variation measure. The method includes discarding the image unit values of the first set of image units. The method also includes generating a second distribution centered at the centroid measure. The method includes randomly sampling the second distribution to obtain new image unit values for the first set of image units. Furthermore, the method includes adding the new image unit values to the first set of image units to obtain a new MR image. The method includes processing the new MR image to identify a location of one or more objects within the subject. The MR image includes one or more additional sets of image units, and the set of image units and the one or more additional sets of image units are non-overlapping. The method further comprises: repeating steps (b) to (g) for each of the one or more additional sets of image units, and adding the respective new image unit values for each of the one or more additional sets of image units to obtain the new MR images.

A better understanding of the nature and advantages of embodiments of the present invention may be gained with reference to the following detailed description and the accompanying drawings.

### BRIEF DESCRIPTION OF THE DRAWINGS

**FIG. 1** shows an example architecture of a machine learning model according to embodiments of the present invention.
**FIG. 2** shows image sub-window processing at inference time according to embodiments of the present invention.
**FIG. 3** shows model inferences of radioactive seeds and isodose contours according to embodiments of the present invention.
**FIG. 4** shows an example architecture of a machine learning model according to embodiments of the present invention.
**FIG. 5** shows a representation of a bounding box model construction according to embodiments of the present invention.
**FIG. 6** shows a workflow for processing outlier voxels according to embodiments of the present invention.
**FIG. 7** is a flowchart of an example process associated with methods for identifying objects in MR images according to embodiments of the present invention.
**FIG. 8** illustrates using multiple machine learning models to identify an object according to embodiments of the present invention.
**FIG. 9** is a flowchart of an example process associated with methods for identifying objects in MR images according to embodiments of the present invention.
**FIG. 10** illustrates a measurement system according to embodiments of the present invention.
**FIG. 11** illustrates a computer system according to embodiments of the present invention.

### DETAILED DESCRIPTION

In brachytherapy, computed tomography (CT) is conventionally used for post-implant imaging. CT can identify locations of radioactive seeds. However, CT is not well suited to identifying locations of soft tissue near implanted radioactive seeds. For example, with prostate cancer, brachytherapy involves radioactive seeds implanted in the prostate. Radiation should be confined to the prostate and should not extend to neighboring organs, such as the rectum. Significant radiation deposition into other organs may result in negative medical outcomes. Thus, distinguishing between soft tissues, such as the prostate and the rectum, is important to high quality brachytherapy.

MRI has high soft tissue contrast. However, radioactive seeds, which may be titanium, stainless steel, or other metals, may appear as negative signal voids. The negative signal voids may appear as vessels, spacers, strand material, or artifacts, and therefore identifying the negative signal voids as radioactive seeds with MRI is difficult. A dosimetrist can spend hours manually localizing radioactive seeds in postoperative images.

Methods that accurately and efficiently locate object (e.g., radioactive seeds) are desired so that images may be used to plan radiosurgery or to assess the success of surgery. MRI-assisted radiosurgery (MARS) can be used for prostate cancer brachytherapy. A dosimetrist interpreting MR images during radiosurgery can be time consuming, expensive, and impractical. Computer techniques can be used to identify objects in images. Techniques using deep machine learning (DL) have been proposed for machine segmentation and localization of the prostate and radioactive seeds in MR images. These DL techniques may still require time-intensive confirmation from a dosimetrist.

Methods and systems described herein improve the quality, accuracy, consistency (e.g., precision), and efficiency for locating objects in MR images. Data from MR images may be pre-processed to remove voxels that are outliers from the rest of the distribution and replace the voxels with voxels with values from within a certain distribution. MR image analysis may also use several models rather than be limited to only one model or a few models. Each voxel may be evaluated for the probability of corresponding to an object by each of several models. These probabilities of a single voxel may be combined to determine an overall probability for the voxel. A particular object within the image may be identified using the overall probabilities for the voxels of the image.

### I. MACHINE LEARNING TECHNIQUES FOR ANALYZING MR IMAGES

Locations of radioactive seeds and organs are useful for brachytherapy. Although the prostate is an organ of interest for radiation treatment planning and post-implant assessment in LDR prostate brachytherapy, it is also beneficial to know the boundary locations for the organs surrounding the prostate to quantify the radiation dosimetry to these structures. From the radiation treatment planning perspective, it is important to minimize the dose to the surrounding normal anatomy. From the post-implant assessment perspective, it is important to quantify the actual radiation dose delivered to the prostate to confirm adequate delivery of the prescribed dose; in the event that the patient is underdosed, the patient may need to return to the operating room to receive additional implants. In addition, it is necessary to compute the dose to normal structures so that toxicity and morbidity can be estimated, allowing for proper patient risk stratification and management after implantation.

### A. Machine learning model architecture for voxels

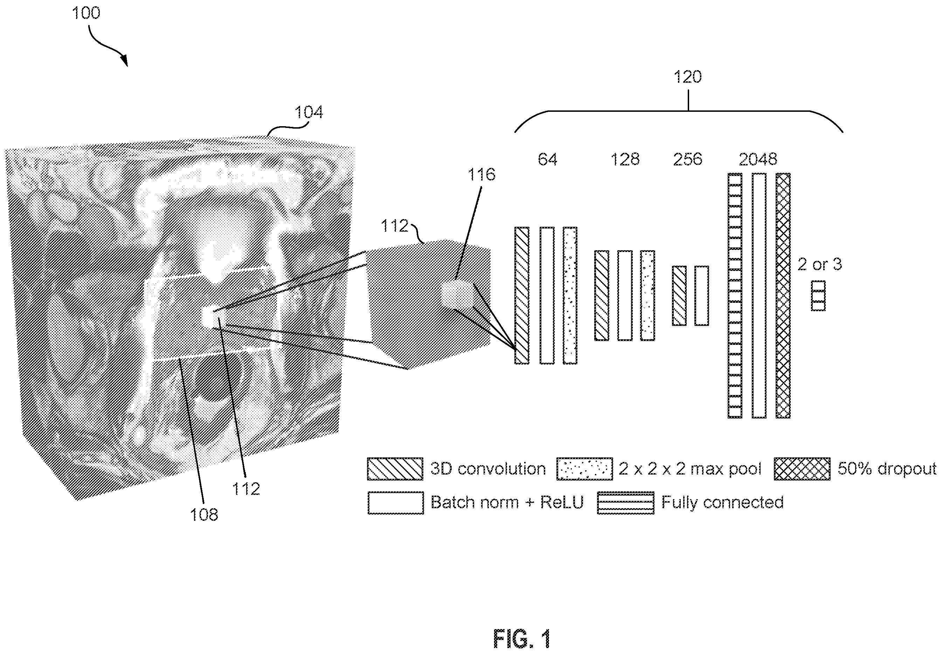

**FIG. 1** shows an example of the architecture 100 of a machine learning model used to analyze MR images. A 3D MR image 104 can be obtained by any suitable MRI scanning equipment. The 3D MR image is composed of voxels. Each voxel is associated with a value that represents the intensity of the image. For a grayscale image, the intensity may be how black or how white the voxel is. For a color image, the intensity may be the RGB (red, green, blue) values or other color model values. The intensity may be represented on any arbitrary scale. For example, the intensity may be a value between 0 and 1, 0 and 10, 0 and 100, 0 and 255, 0 and 4095, or 0 and 65,535. FIG. 1 involves a machine learning model architecture that may be applied for locating radioactive seeds in voxels of an MR image.

MR image 104 may include a region of interest (ROI) 108. ROI 108 is a 3D region composed of voxels. In this example, ROI 108 includes the prostate. This example involves the identification of radioactive seeds rather than an organ, such as the prostate. The identification of the prostate by the machine learning model is therefore not essential in this example. But other examples can identify an organ (e.g., the prostate) using embodiment described herein. However, in this example, the prostate may be identified by a human (dosimetrist, radiologist, or other medical professional) or by a machine learning model or may not need to be identified. In some embodiments, ROI 108 may be determined by bounding box detection described elsewhere in this disclosure.

A machine learning model may use sliding windows to analyze 3D MR image 104. A sliding window involves taking a window of a fixed size and performing analysis with that window. The window is then "slid" to another area that may or may not overlap with the previous window, and analysis is repeated. The sliding of the window and the analysis in the slid window are repeated until ROI 108 is covered by all sliding windows. Windows may be slid over by the same amount. Section 112, which is a section of ROI 108, is magnified for clarity. Section 112 includes window 116. Window 116 is one of many sliding windows in ROI 108, with several windows overlapping with window 116. Each window may include a plurality of voxels. In some embodiments, section 112 or window 116 may be determined by bounding box detection described elsewhere in this disclosure.

Each window 116 can be analyzed by a model, e.g., as shown in FIG. 1. In the example of FIG. 1, a convolutional neural network (CNN) 120 is used. In order from left to right, the model includes three 3D convolutional layers, e.g., with 3 × 3 × 3 convolution kernels. Maximum pooling (2 × 2 × 2, non-overlapping) followed by batch normalization and rectified linear unit activations were used between the convolutional layers. The third convolutional layer was connected to a fully connected layer with 2048 neurons. The output layer was a fully connected layer with 2 or 3 neurons, depending on the inference task. Other CNN configurations may also be used.

For this example, the network was constructed by starting with a single convolutional layer, a fully connected layer, and an output layer, and then the number of convolutional layers was increased until the accuracy on the cross-validation windows plateaued at a maximum value. Cross-validation windows involve holding out a portion of the data to quantify the accuracy of the algorithm as the model is training. Once the accuracy on the validation windows plateaus, the training may be terminated.

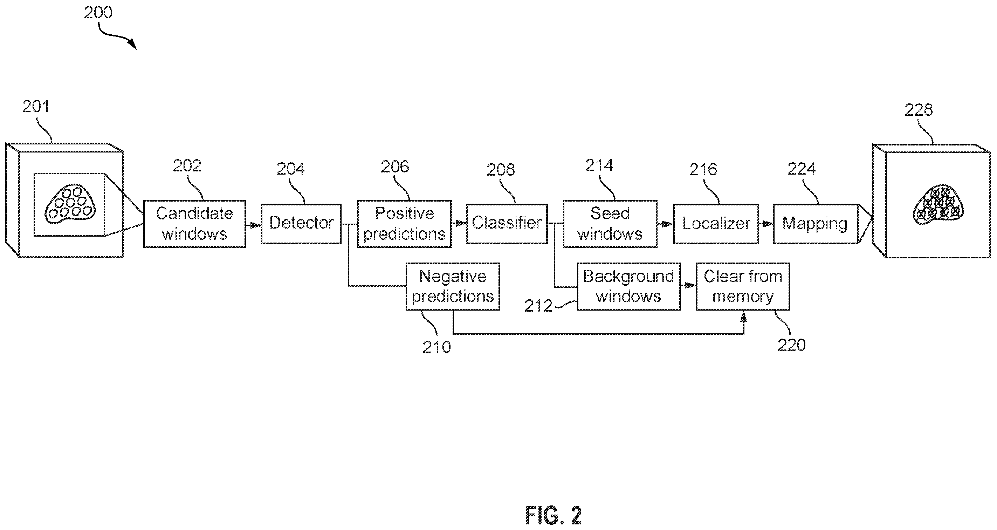

**FIG. 2** summarizes the analysis 200 using CNN 120 of FIG. 1 on windows of an MR image 201. Each window 202 was passed to a detector 204 to identify windows containing a particular object (radioactive seeds in this example). Some windows were identified by detector 204 as containing seeds (positive predictions 206). Windows identified by detector 204 as not containing seeds (negative predictions 210) are cleared from memory at stage 220. Positive predictions 206 were then passed to a classifier 208 to reject potential false positives. For example, classifier 208 rejects seed marker windows that, owing to their similar shape and proximity to seeds, were incorrectly classified as seed windows and were then classified as background windows 212. Seed windows 214 from classifier 208 were passed to a localizer 216 to pinpoint the precise location of the seed within the window. Finally, the seed windows were mapped at stage 224 back to their locations within the original image stack. The result was image 228 showing locations of seeds. Detector 204, classifier 208, and localizer 216 each are a CNN and were all constructed with the same configuration of layers as shown in FIG. 1. More than one seed window may correspond to a particular seed. A given seed may have multiple voxels, with each voxel having a separate prediction for probability of corresponding to a seed.

In this example, two neurons (background, seed) were used in the output layer for detector 204. Classifier 208 had 3 neurons (background, seed, seed marker) in the output layer. Localizer 216 had 3 neurons (1 for each of the x, y, and z coordinates) in the output layer to estimate the seed location within the sub-window.

Two fully convolutional variants of the architecture shown in FIG. 1 were tested. The first replaced the dense fully connected layer with a 3D convolutional layer using a 3 × 3 × 1 kernel and no zero padding. The second replaced the dense fully connected layer with a 3 × 3 × 1 average pooling layer followed by a 3D convolutional layer using a 1 × 1 × 1 kernel. The dense fully connected layer resulted in the lowest loss on the validation data set, being two orders of magnitude and one order of magnitude lower than the fully convolutional approaches, respectively.

Machine learning models may be trained using a set of training MR images. The training MR images may include images with objects identified by a dosimetrist. Hence, voxels corresponding to an object and not corresponding to an object are known. The machine learning models then identify features to distinguish voxels corresponding to objects and not corresponding to objects. Objects may include radioactive seeds, organs, tissues, or other objects of clinical interest. A "tissue" corresponds to a group of cells that group together as a functional unit. More than one type of cells can be found in a single tissue.

### B. Machine learning model results

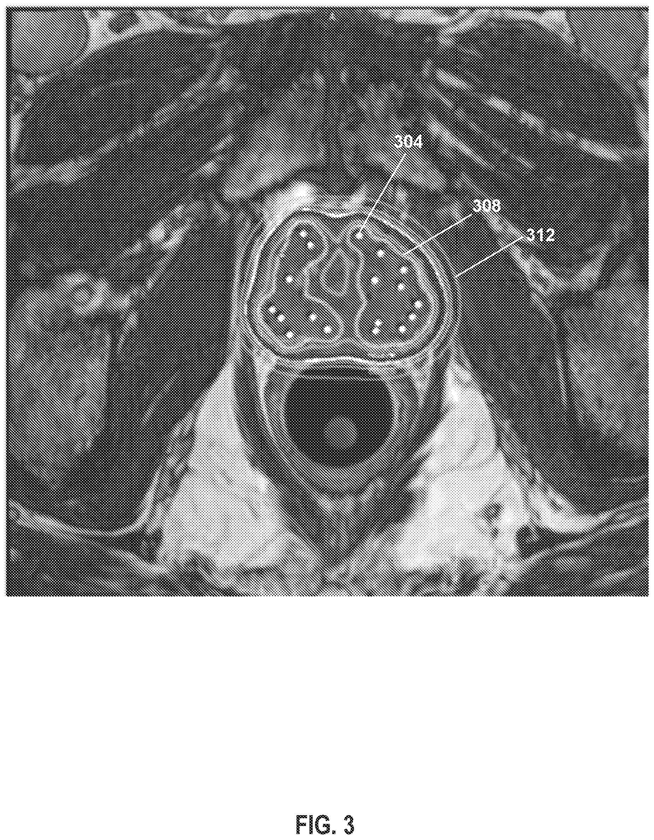

**FIG. 3** shows model inferences of radioactive seeds and isodose contours. The contours of FIG. 3 may be determined through the model described with FIGS. 1 and 2. Radioactive seeds (shown as yellow circles) are identified. An example of a radioactive seed is seed 304. The contours show radiation dose distributions based on the identified radioactive seeds. Each contour is an isodose line. Contour 308 is closest to the radioactive seeds and shows the highest dose. Contour 312 is farthest from the radioactive seeds and shows the lowest dose. The contour for the prostate organ may or may not be determined by the model but is not shown in FIG. 3 for clarity of the isodose lines. The isodose lines are important for determining that the prostate receives therapeutic radiation, while other tissues (e.g., rectum) receive minimal or acceptable levels of radiation.

### C. Machine learning model architecture for pixels

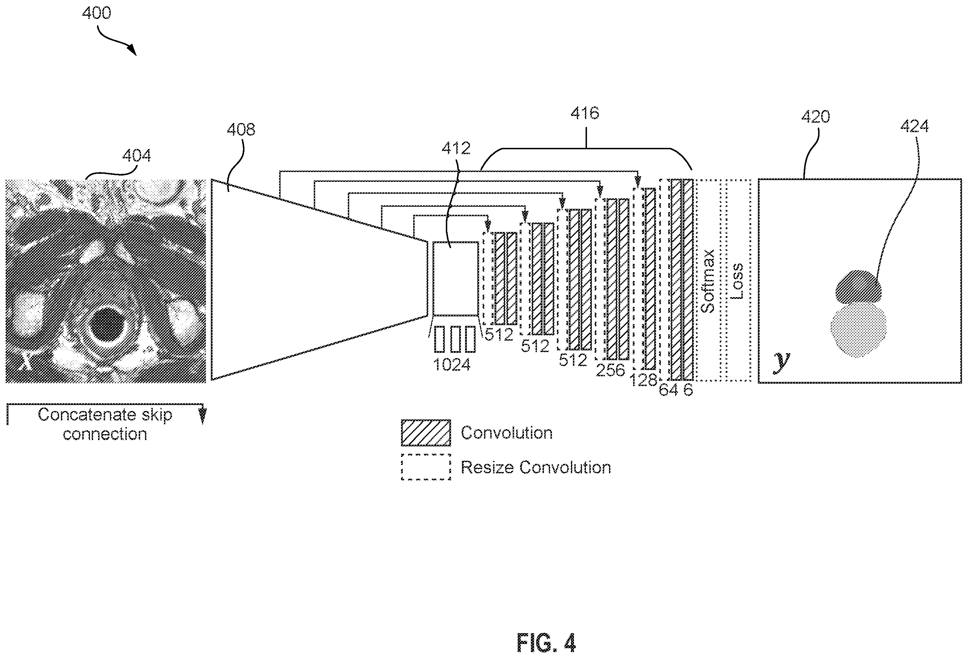

**FIG. 4** shows an example of an architecture 400 of a machine learning model used to analyze pixels from MR images. A 2D MR image 404 can be obtained by any suitable MRI scanning equipment. The 2D MR image may be obtained by taking a two-dimensional slice of a 3D MR image with voxels. The 2D MR image includes pixels instead of voxels.

Convolutional encoder 408 processes 2D MR image 404. Convolutional encoder 408 may be a fully convolutional network (FCN). Convolution encoders may include VGG 16 and 19 (stacked convolutions); DenseNet 121, 169, and 201 (densely connected convolutions); ResNet 50, 50V2, 101, 101V2, 152, and 152V2 (residual connections); ResNeXt 50 and 101 (split-transform-aggregate residual connections); Inception V3 (inception modules); InceptionResNet V2 (inception modules and residual blocks); Xception (xception modules); and other suitable modules or encoding functions.

Latent space 412 may result from the encoder. Latent space 412 may be three stacked convolutions with 424 feature maps each or other suitable convolutions with feature maps.

Convolutional decoder 416 may decode the latent space representation back to the original image size. The convolutional decoder may include stacked convolutions and resize convolutions. Skip connections may be made between the convolutional encoder 408 and convolutional decoder 416 at equivalent resolution scales.

Output image 420 shows organ segmentation resulting from the machine learning model. Region 424 shows an area determined to correspond to the prostate. After the convolutional decoder 416, each pixel may be associated with a vector of probabilities corresponding to an organ or background. For example, the vector may be (pₐ, p_{b}, p_{c}, p_{d}, pₑ, p_{f}), where p refers to the probability that the pixel corresponds to one of five organs (e.g., prostate, rectum, seminal vesicle, external urinary sphincter, or bladder) or background. The probabilities may sum to 1.

Architecture 400 and architecture 100 may be used together on image units resulting from the same MRI scan to identify radioactive seeds and to identify organs.

### D. Bounding box detection

In some embodiments, bounding boxes surrounding an object may be detected. The object may be a radioactive seed or an organ. The bounding box may be detected by a single-shot detector (SSD). After the bounding box is detected, organ segmentation or seed localization may be performed in the bounding box. Although the term "box" is used, other shapes can be used, including circles, ellipses, and polygons.

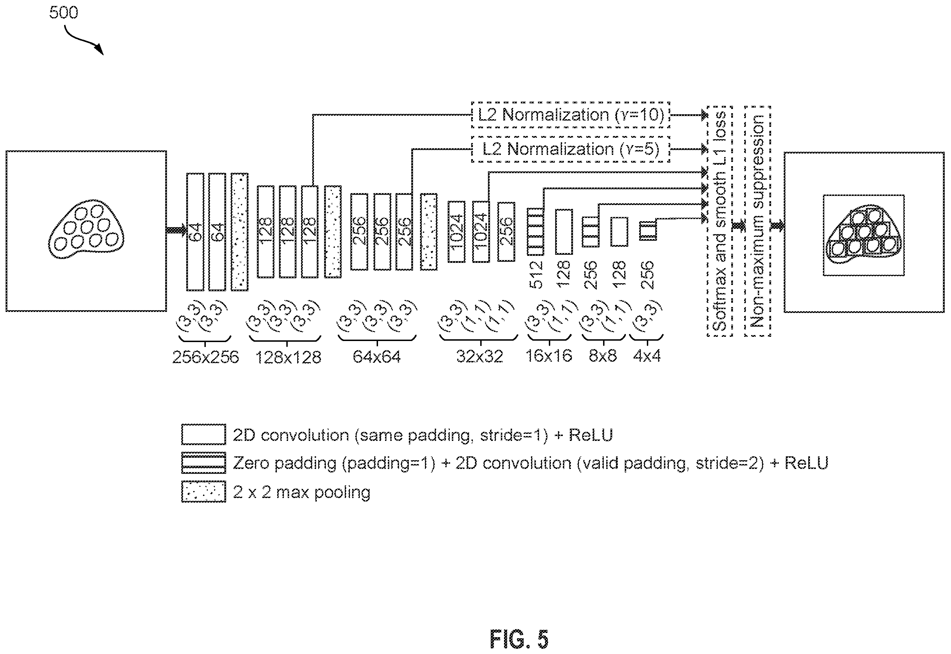

As an example, the SSD may be constructed using the Keras DL library with a fully convolutional network (FCN) backbone including 16 stacked convolutional layers. The resolutions of the feature maps may range from 256 × 256 to 4 × 4 with a downsampling factor of 2 used for each downsampling step. Prediction heads may be constructed at six resolution scales, including 128 × 128, 64 × 64, 32 × 32, 16 × 16, 8 × 8, and 4 × 4. L2 normalization layers may be applied after the 128 × 128 and 64 × 64 feature maps to normalize the scales. The SSD loss function, including a summation of classification and regression losses, may be used to train the network. The SSD outputs may be bounding box coordinates surrounding potential seeds or organs and the probability that the box was classified as including a seed or organ (e.g., value between 0 and 1). **FIG. 5** shows a representation 500 of an SSD model construction. A unique aspect of SSD is the ability to predict multiple objects of different sizes within the same image, which may be beneficial for brachytherapy because the size, location, and number of seeds may vary depending on patient and treatment. SSD may also predict the location of the prostate or any organ described herein. The predictions from the SSD may be an intermediate output to a machine learning model that performs seed localization (e.g., as described with FIGS. 1-3) or organ segmentation (e.g., as described with FIG. 4).

The output of bounding box detection may include the box coordinates of the vertices and a value representing the object class (e.g., seed, type of organ). Bounding boxes can be applied in two dimensions or three dimensions.

Embodiments involving bounding box coordinate detection are described elsewhere in this application. Bounding box coordinate detection may be similar to methods described in Sanders, J.W., "Development of fully balanced SSFP and computer vision applications for MRI-assisted radiosurgery (MARS)," Ph.D. dissertation, University of Texas (May 2020) and Zhou et al., "MetNet: Computer-aided segmentation of brain metastases in post-contrast T1-weighted magnetic resonance imaging," Radiotherapy and Oncology (December 1, 2020) (doi:10.1016/j.radonc.2020.09.016).

### II. MRI PRE-PROCESSING

MRI data may include image units (e.g., voxels or pixels) with outlier values. These outliers may be from noise related to equipment limitations (e.g., inhomogeneities in the magnetic field), imaging anomalies (e.g., artifacts), or expected statistical variation (e.g., subject-to-subject variation). These outlier image units may skew analysis by machine learning models. Simply removing these image units from analysis may also skew results by unintentionally weighting nearby image units more in analysis compared image units farther away from these outlier image units. Embodiments described herein address the outlier image units.

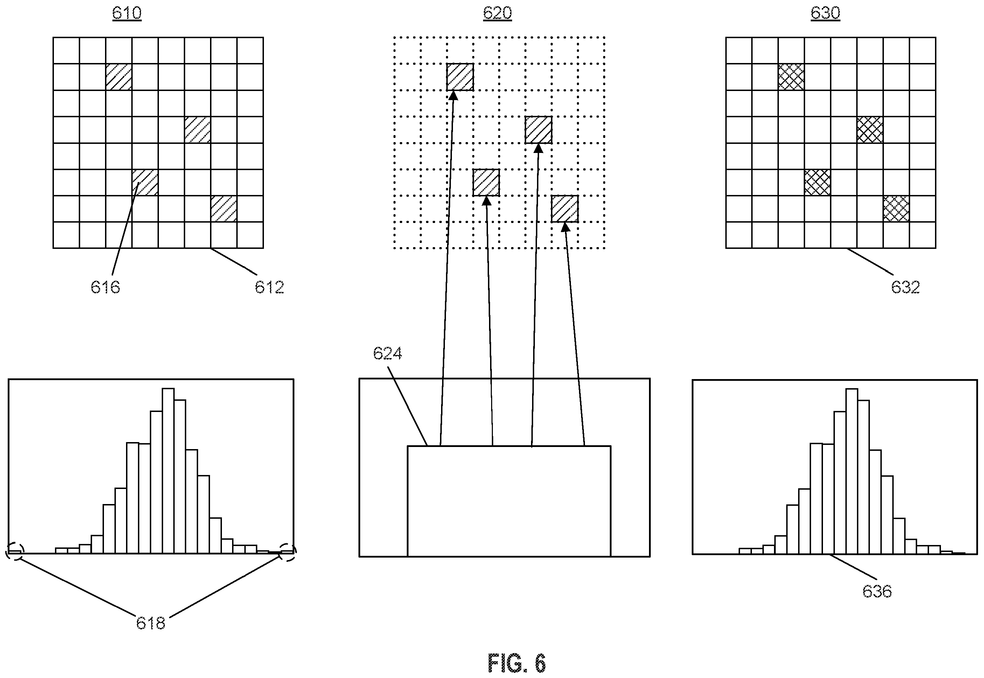

**FIG. 6** illustrates a workflow for processing outlier image units. At stage 610, window 612 may include a plurality of voxels. Window 612 is illustrated as two-dimensional for simplicity but could be a three-dimensional window (e.g., for voxels). Additionally, window 612 may include more or fewer image units than shown in the figure. Some of the image units in window 612 may be outliers. The four shaded image units in window 612, including image unit 616, are outliers. The outlier image units can correspond to columns 618 in the histogram of image unit values. The histogram may be a histogram of image unit values for the entire MR image, a region of interest, or the window. As examples, the distribution of image unit values may be a Rician distribution or a normal distribution. In some examples, the distribution may be a Rayleigh, Central Chi, Noncentral Chi, Central Chi Square, Noncentral Chi Square, Gamma, Folded Normal, or Half-Normal distribution, or any combination of distributions disclosed herein.

At stage 620, a random distribution 624 is generated. Random distribution 624 may have the same mean (or other statistical value, such as median) as the histogram in stage 610. In some embodiments, a random number is generated. The random number may then be multiplied by the mean (or other statistical value such as the median) to generate the random distribution. In other embodiments, a random number may be generated in a range centered on the mean (or other statistical value). The range of the random number may be limited to a certain distance from the mean or other statistical value. For example, the random number may not exceed one, two, or three standard deviations or may be within a certain percentile range. Random distribution 624 may be randomly sampled to provide values to replace the outlier image units.

At stage 630, the outlier image units have been replaced with values from random distribution 624 to form window 632. The outlier image units may be randomly sampled from the distribution. The replacement values can be adjusted so that the mean (or other statistical value) is the same as the mean of random distribution 624 (and also mean of the histogram in stage 610). Histogram 636, associated with window 632, no longer shows columns 618 associated with the outlier image units. The values of the histogram may be scaled to a range, such as [0, 1] after outliers have been replaced.

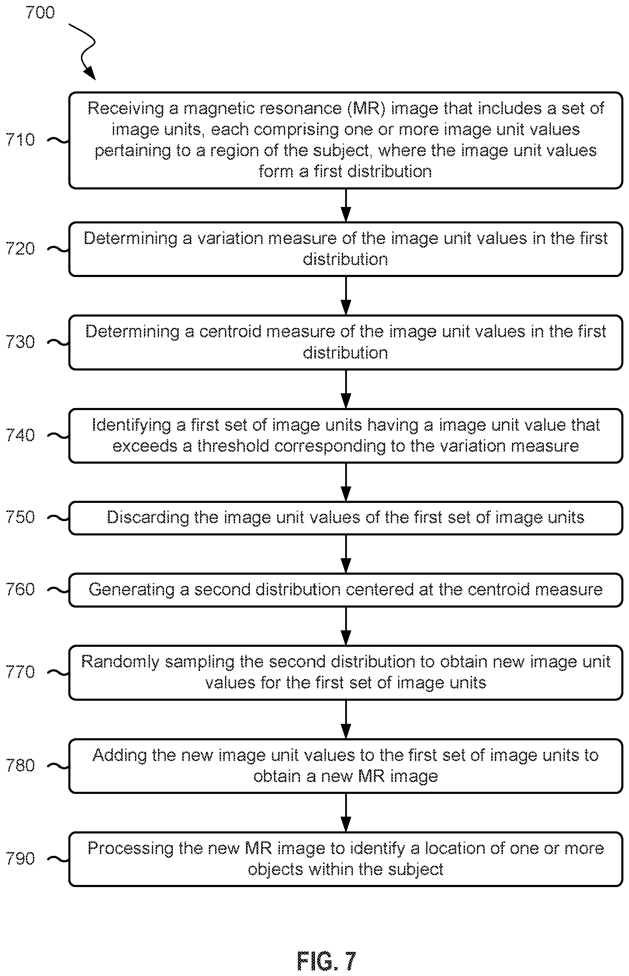

**FIG. 7** is a flowchart of an example process 700 associated with methods for identifying objects in MR images, including the method described with FIG. 6. In some implementations, one or more process blocks of FIG. 7 may be performed by a system (e.g., system 1000). In some implementations, one or more process blocks of FIG. 7 may be performed by another device or a group of devices separate from or including the system. Additionally, or alternatively, one or more process blocks of FIG. 7 may be performed by one or more components of system 1000, such as imaging device 1010, emitter 1008, detector 1020, logic system 1030, memory 1035, external memory 1040, storage device 1045, processor 1050, and/or treatment device 1060.

At block 710, an MR image is received that includes a set of image units. The image units may be pixels or voxels. The set of image units may be a two-dimensional set of pixels or a three-dimensional set of voxels. Each set of image units includes one or more image unit values pertaining to a region of the subject. For example, the three-dimensional set of image units may be ROI 108 or MR image 104 of FIG. 1 or window 612 in FIG. 6. The image unit values form a first distribution. For example, the image unit value may be an intensity of the image unit, with 0 being black and 4095 being white (or 0 being white and 4095 being black). The first distribution may be represented by histogram displaying frequency on the y-axis and intensity on the x-axis. In some embodiments, the image unit values may be normalized values from the MR image (e.g., normalized by a maximum intensity).

At block 720, a variation measure of the image unit values in the first distribution is determined. Determining the variation measure of the image unit values in the first distribution may include determining the standard deviation of the image unit values in the first distribution. In some embodiments, determining the variation measure of the image unit values may include determining a percentile or percentiles of the first distribution. For example, determining the variation measure may include determining the values corresponding to 1, 2, 3, 4, 5, 6, 7, 8, 9, 10, 15, 20, 80, 85, 90, 91, 92, 93, 94, 95, 96, 97, 98, and/or 99 percentiles.

At block 730, a centroid measure of the image unit values in the first distribution is determined. The centroid measure may be the mean or median of the first distribution.

At block 740, a first set of image units having an image unit value that exceeds a threshold corresponding to the variation measure is identified. The threshold may be equal to the variation measure. In some embodiments, the threshold may be proportional (e.g., a multiple) of the variation measure. For instance, the threshold may be two or three times the standard deviation. As an example, image units corresponding to columns 618 in FIG. 6, including image unit 616, may be image units that exceed a threshold.

At block 750, the image unit values of the first set of image units may be discarded. In a histogram representation of the first distribution, the discarded image unit values may be the tails and ends of the histogram. Discarding these outlier image unit values may reduce errors in analysis. Analysis in regions near an outlier image unit value may be skewed by the outlier image unit value.

At block 760, a second distribution centered at the centroid measure may be generated. The second distribution may be a random distribution. The random distribution may be generated with a computer. In some embodiments, the random distribution may be generated without a computer or without mathematical calculations by flipping a coin, rolling a die or dice, selecting among different tickets (e.g., a lottery), or performing other physical operations. The second distribution may be chosen or modified such that the second distribution does not exceed the threshold. In some embodiments, the second distribution may be generated to have the same number of values as the number of discarded image unit values. Random distribution 624 is an example of a second distribution.

At block 770, the second distribution may be randomly sampled to obtain new image unit values for the first set of image units. In some embodiments, sampled image unit values may be compared to the threshold, and if the sampled image unit value exceeds the threshold, the second distribution may be sampled again to obtain new image unit values that are within the threshold. In some embodiments, a centroid measure of the sampled image unit values is determined. A separation value between centroid measure of the sampled image unit values and the centroid measure of the image unit values in the first distribution may be determined. The separation value may be a difference or a ratio. If the separation value is greater than a cutoff value, the second distribution may be randomly sampled again until the separation value is less than the cutoff value. As examples, the separation value may be within 5%, 10%, 15%, 20%, or 25% of the centroid measure of the image unit values in the first distribution. In some embodiments, all elements of the second distribution may be used to replace the discarded image unit values.

At block 780, the new image unit values are added to the first set of image units to obtain a new MR image. Image units that are outliers are replaced with image units that have a centroid measure equal to or close to the centroid measure of the image unit values in the first distribution. Image units may be replaced randomly with values from the second distribution. Window 632 is an example showing image units replaced with values from the second distribution.

At block 790, the new MR image may be processed to identify a location of one or more objects within the subject. The one or more objects may include one or more radioactive seeds. In some embodiments, the one or more objects may include markers, which may be adjacent to a seed. In some embodiments, the one or more objects may include an organ or the boundary of an organ. The organ may be a prostate, rectum, seminal vesicle, external urinary sphincter, bladder, or any organ described herein. The one or more objects may also include a neurovascular bundle, bladder neck, prostate base, prostate apex, prostate peripheral zone, prostate central zone, anterior rectal wall, or any other object that may be contained within an MR image of the prostate. Processing the new MR image may occur before radiosurgery to plan placement of radioactive seeds among organs or after radiosurgery to assess the placement of radioactive seeds among the organs. Processing the MR image may be by any method described herein, including the methods described with FIGS. 1, 2, 3, 4, and 7.

Processing the new MR image may include determining coordinates of a bounding box around a radioactive seed or organ. Determining coordinates of a bounding box may include using a single-shot detector (SSD) as described herein and shown in FIG. 5. The bounding box may be a polygon, including a square, a rectangle, or a triangle. The coordinates of the bounding box may be the coordinates of the vertices, the lines, or any other parts of the polygon that help define the boundaries of the bounding box. In some embodiments, the bounding box may be a circle or ellipse, and the coordinates may include centers, radii, and/or axis lengths. The bounding box may then provide a smaller region to be processed to locate the radioactive seed or to identify an organ or boundary of an organ. The bounding box may be used in three dimensions, where the box may be a sphere, spheroid, or prism, including a cube, rectangular solid, or pyramid,

Process 700 may include calculating radiation values for a subset of the set of image units. The radiation values may be calculated based on the identified locations of the one or more radioactive seeds. For example, contour lines showing isodoses, as in FIG. 3 may be generated. Process 700 may include implanting additional radioactive seeds into the subject. Implanting additional radioactive seeds may be after determining a low or suboptimal radiation value for an image unit or a subset of image units. For example, the radiation value at a specific location or locations may be lower than a threshold value. Implanting additional radioactive seeds may occur while the subject is being imaged by an MRI scanner. After implanting additional radioactive seeds, process 700 may be repeated again to identify the location of the new seed(s) and to calculate radiation values associated with the new seed configuration. Additional seeds may be implanted based on the new radiation values. In some embodiments, implanted seeds may be removed based on the radiation values. For example, the radiation value at a specific location or locations may be higher than a second threshold value. In some embodiments, the location of an implanted seed may be changed based on the isodose contour lines.

In some embodiments, local neighborhoods will be used for replacing outliers instead of the entire image. The MR image includes one or more additional sets of image units. The set of image units and the one or more additional sets of image units are non-overlapping. The sets of image units may be window 116 and additional windows. In some embodiments, the local neighborhoods may be determined by bounding box detection. The process includes repeating blocks 720 to 770 for each of the one or more additional sets of image units. The respective new image unit values for each of the one or more additional sets of image units are added to obtain the new MR images.

Process 700 replaces outlier image unit values with image unit values sampled from a distribution having a centroid measure equal to the centroid measure of image unit values in another distribution. Surprisingly, process 700 results in more accurate and efficient identification of particular objects in MR images. The improved results are unexpected because one of skill in the art would not predict that a random replacement of values would improve identification. The random replacement in process 700 imitates noise in MRI. As a result, the new image unit values will not bias the analysis of the image and the identification of objects.

Process 700 may include additional implementations, such as any single implementation or any combination of implementations described and/or in connection with one or more other processes described elsewhere herein.

Although FIG. 7 shows example blocks of process 700, in some implementations, process 700 may include additional blocks, fewer blocks, different blocks, or differently arranged blocks than those depicted in FIG. 7. Additionally, or alternatively, two or more of the blocks of process 700 may be performed in parallel.

### III. MRI POST-PROCESSING

Multiple machine learning models may be available to identify objects in an MR image. Each machine learning model is limited in the accuracy in identifying and locating an object. Many machine learning models calculate a probability of an object being present. The user or the model may set a minimum probability for determining that the object is present (i.e., that a given voxel is within the object). Using multiple models can improve the identification and localization of an object in an MR image. For example, if one model determines the probability that the voxel is within an object is 0.9, there is higher confidence of the object being present when a second model determines the probability to be 0.9 compared to when the second model determines the probability to be 0.5.

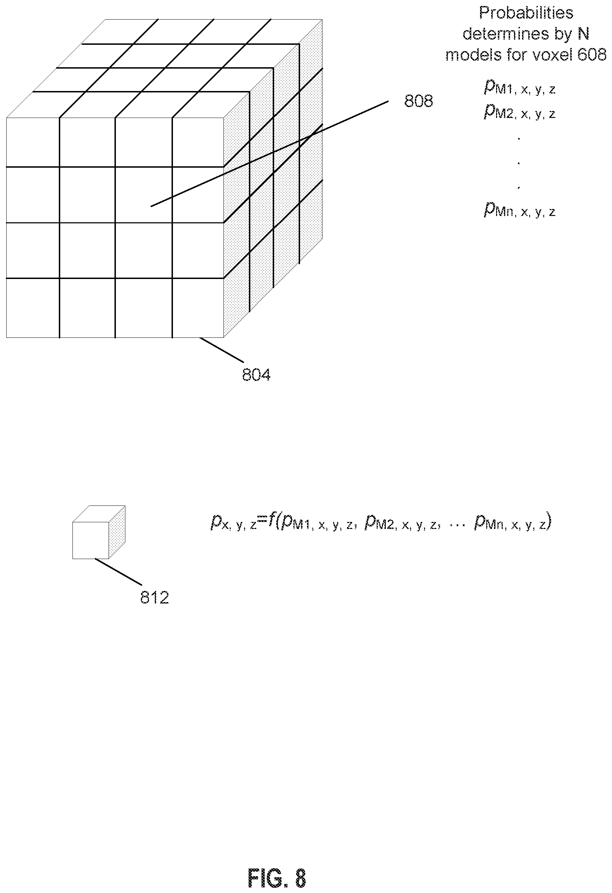

**FIG. 8** illustrates how multiple machine learning models may be used to identify an object. Region 804 is a three-dimensional volume of voxels, including voxel 808. Each voxel in region 804 may be analyzed by *n* machine learning models: M1, M2, ... Mn. The probability of voxel 808 corresponding to a particular object determined by model M1 is denoted by *p*_{M1}, _{x, y, z}, where (x, y, z) provide the coordinates of the voxel. The probability of voxel 808 corresponding to a particular object determined by model Mn is *p*_{Mn, x, y, z}. As each model calculates a probability of each voxel in region 804, each model includes a matrix of probabilities with each entry corresponding to a different voxel with different coordinates.

The probability of a voxel may be determined based on probabilities calculated from all *n* machine learning models. For example, the probability of voxel 812 corresponding to a particular object may be *p*_{x, y, z}, which may be a function of the probabilities of each of the *n* machine learning models. As examples, the function may be an average, a weighted average, or some other combination of the individual model probabilities. Although FIG. 8 shows an example with voxels, a similar process can be used with pixels.

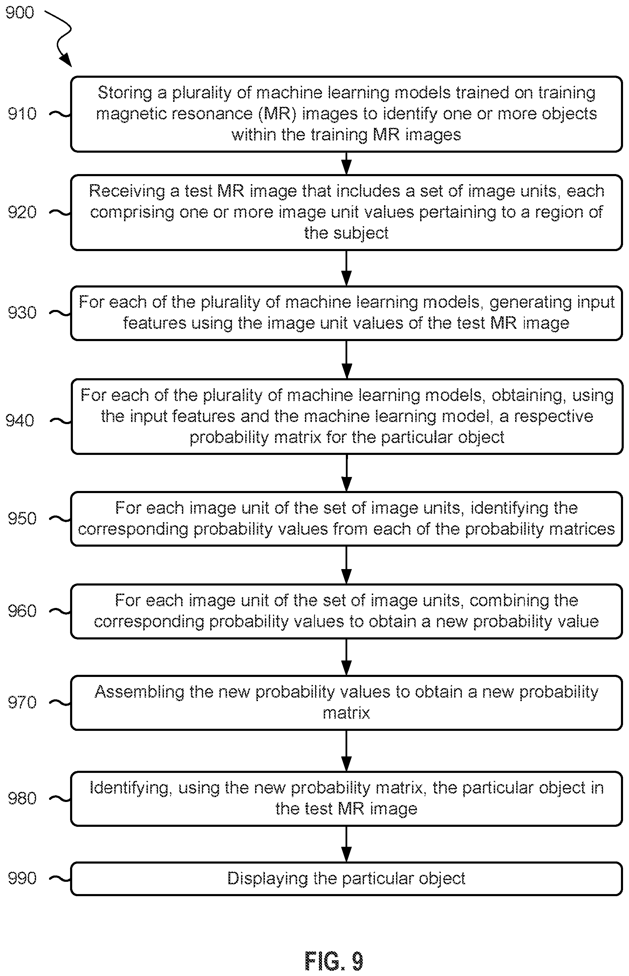

**FIG. 9** is a flowchart of an example process 900 associated with methods for identifying objects in MR images, including the method described with FIG. 8. In some implementations, one or more process blocks of FIG. 9 may be performed by a system (e.g., system 1000). In some implementations, one or more process blocks of FIG. 9 may be performed by another device or a group of devices separate from or including the system. Additionally, or alternatively, one or more process blocks of FIG. 9 may be performed by one or more components of system 1000, such as imaging device 1010, emitter 1008, detector 1020, logic system 1030, memory 1035, external memory 1040, storage device 1045, processor 1050, and/or treatment device 1060.

At block 910, a plurality of machine learning models trained on training MR images to identify one or more objects within the training MR images is stored. Each training MR image includes image units. Image units may be pixels or voxels. A set of pixels is two-dimensional, and a set of voxels is three-dimensional. Each training MR image includes one or more image unit values pertaining to a region of a training subject. Each of the plurality of machine learning models provides a probability matrix of probability values. Each probability value may provide a probability that a given image unit includes a particular object within the subject. The particular object may be an organ or a radioactive seed, including any organ or radioactive seed described herein.

A machine learning model may be similar to the model described with FIG. 1 or FIG. 4. The model may include a supervised learning model. Supervised learning models may include different approaches and algorithms including analytical learning, artificial neural network, backpropagation, boosting (meta-algorithm), Bayesian statistics, case-based reasoning, decision tree learning, inductive logic programming, Gaussian process regression, genetic programming, group method of data handling, kernel estimators, learning automata, learning classifier systems, minimum message length (decision trees, decision graphs, etc.), multilinear subspace learning, naive Bayes classifier, maximum entropy classifier, conditional random field, Nearest Neighbor Algorithm, probably approximately correct learning (PAC) learning, ripple down rules, a knowledge acquisition methodology, symbolic machine learning algorithms, subsymbolic machine learning algorithms, support vector machines, Minimum Complexity Machines (MCM), random forests, ensembles of classifiers, ordinal classification, data pre-processing, handling imbalanced datasets, statistical relational learning, or Proaftn, a multicriteria classification algorithm The plurality of machine learning models may also include other machine learning models, including convolutional neural networks (CNN), linear regression, logistic regression, deep recurrent neural network (e.g., long short-term memory, LSTM), Bayes classifier, hidden Markov model (HMM), linear discriminant analysis (LDA), k-means clustering, density-based spatial clustering of applications with noise (DBSCAN), random forest algorithm, and support vector machine (SVM).

The plurality of machine learning models may include at least 2, 3, 4, 5, 6, 7, 8, 9, 10, 20, 30, 40, 50, 60, 70, 80, 90, 100, 110, or more than 110 models. A certain number of models may result in computational efficiency, where adding the last model increases the accuracy of the model by the largest amount and adding another model would increase the accuracy of the model by a smaller amount. The number of models for computational efficiency may be 7, 8, 9, 10, or in a range from 5 to 10, 10 to 15, 15 to 20, or 20 to 30. In some embodiments, a certain minimum number of models may converge onto the same result, regardless of the identity of the machine learning model. The minimum number of models for convergence may be 80 to 90.

At block 920, a test MR image that includes a set of image units is received. Each test MR image includes one or more image unit values pertaining to a region of the subject. The test MR image may be pre-processed, as explained with FIG. 7. For example, process 900 may include receiving an MR image that includes a set of image units. Each MR image may include one or more image unit values pertaining to a region of the subject, where the image unit values form a first distribution. A variation measure of the image unit values in the first distribution may be determined. A centroid measure of the image unit values in the first distribution may be determined. A first set of image units having an image unit value that exceeds a threshold corresponding to the variation measure may be identified. The image unit values of the first set of image units may be discarded. A second distribution centered at the centroid measure may be generated. The second distribution may be randomly sampled to obtain new image unit values for the first set of image units. The new image unit values may be added to the first set of image units to obtain the test MR image.

At block 930, for each of the plurality of machine learning models, input features using the image unit values of the test MR image are generated. The input features may be determined from the machine learning models. As examples, the input features may be the raw MR image values for each image unit in a window or the image units determined after pre-processing, e.g., using process 700. The input features may include the image unit values, neighboring image unit values (e.g., within a specified number of image units of the particular image unit), derivative of the image unit value to an adjacent image unit, identification of a neighboring image unit, among other input features.

At block 940, for each of the plurality of machine learning models, a respective probability matrix for the particular object may be obtained using the input features and the machine learning model. For example, the probability matrix may include the coordinates of the image units and the probability that each image unit corresponds to the particular object.

At block 950, for each image unit of the set of image units, the corresponding probability values from each of the probability matrices are identified. For example, for a given image unit, the probability value of that image unit corresponding the particular object is identified for each of the probability matrices, with each matrix from a different machine learning model. As an example, a probability matrix for model Mn may include *p*_{Mn, x, y, z} for different image unit coordinates (x, y, z), as depicted in FIG. 8.

At block 960, for each image unit of the set of image units, the corresponding probability values are combined to obtain a new probability value. Combining the corresponding probability values to obtain the new probability value may include calculating the average, mode median, or a percentile (e.g., 10, 20, 30, 40, 60, 70, 80, 90) of the corresponding probability values. The probability value for the image unit may be *p*_{x, y, z}, as described with FIG. 8.

At block 970, the new probability values may be assembled to obtain a new probability matrix. For example, the new probability matrix may include the coordinates of the image units and the new probability value that each image unit corresponds to the particular object. The new probability matrix may include the different values of *p*_{x*,* y, z} for the different voxel coordinates (x, y, z) or the different values of *p*_{x, y} for the different pixel coordinates (x, y).

At block 980, the particular object in the test MR image is identified using the new probability matrix. Identifying the particular object in the test MR image may include comparing the new probability values to a threshold value. The image unit may be assigned to the particular object when the corresponding new probability value is equal to or exceeds the threshold value. The threshold value may be determined from a reference MR image or MR images. Image units in the reference MR image(s) may be assigned to a particular object by a medical practitioner. The threshold value may be a value determined from the image units in the reference MR image(s) that arbitrates between being assigned to the particular object and not being assigned to the particular object.

In some embodiments, sliding windows of image units may be used to identify a particular object. The sliding windows may include a window similar to sub-window 116 in FIG. 1. The set of image units may be a first set of image units. The test MR image may include a plurality of sets of image units. The plurality of sets of image units includes the first set of image units as one of the plurality. Each of the plurality of sets of image units may overlap with at least one other set of image units. The process may include repeating blocks 930 to 980 for each of the plurality of the sets of image units. The boundaries of the particular object may be determined using the identifications of the particular object from the plurality of the sets of image units.

For example, an object may be identified in the first set of image units. The window may be slid over and a second set of image units may be analyzed. The second set of image units may or may not overlap with the first set of image units. The object may also be identified in the second set of image units to confirm the object identified in the first set of image units. In some embodiments, the two different sets of image units may not be in exact agreement. Each image unit result may be average between the two (or more) sets of image units. For example, with three sets of image units, two sets may identify the object at a particular image unit, while one set may identify no object at the particular image unit. Based on the average or the majority vote of the sets, the image unit is identifies as corresponding to the particular object. In some embodiments, a minimum number of sets of image units may be required to identify an object. The plurality of machine learning models may be applied to each sliding window.

Each of the plurality of sets of voxels may have dimensions of *nₓ* by *n_{y}* by *n_{z}* voxels. Each of the dimensions (*nₓ, n_{y}, n_{z}*) may be 3, 4, 5, 6, 7, 8, 9, 10 to 15, 15 to 20, 20 to 25, or more. Each of the plurality of three-dimensional sets of voxels may have the same dimensions. Sets of pixels may have dimensions of *nₓ* by *n_{y}* similar to the dimensions for voxels.

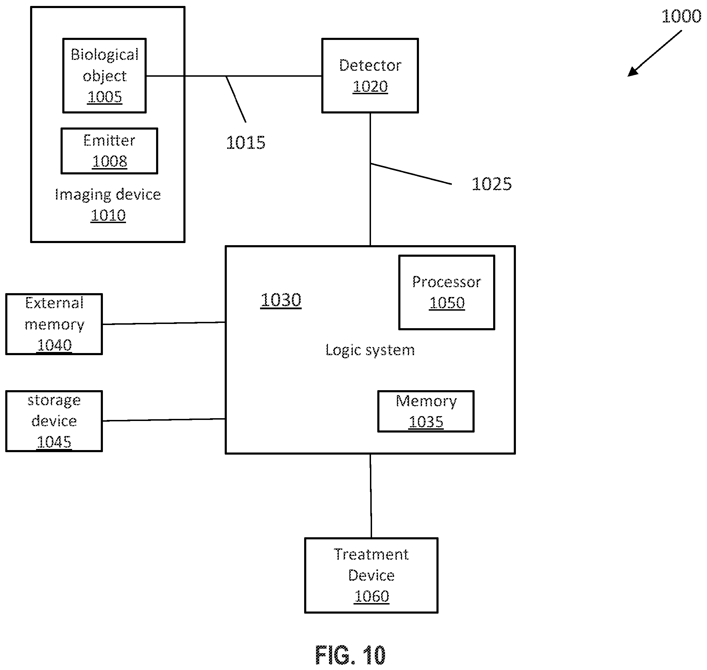

At block 990, the particular object may be displayed. Displaying the particular object may include displaying a border of the particular object. Displaying the particular object may include displaying the locations of radioactive seeds, similar to FIG. 3. Displaying the particular object may also include displaying an organ, including any described herein. The MR image with displayed objects may be further analyzed to understand radiation doses and other clinical implications. Other analysis of the processed MR image may include analyses described with process 700 in FIG. 7. Treatment of the subject may also occur, as described with treatment device 1060 in FIG. 10.

In some embodiments, process 900 may include generating an entropy value for the plurality of machine learning models and determining a confidence interval for the particular object using the entropy value. An entropy value of 0 may correspond to a scenario in which all models agree on a voxel being in a particular object. The maximum entropy for a voxel may correspond to a scenario in which all models select different objects for the particular voxel. The entropy may be computed for a plurality of voxels. The entropy may be a spatial entropy, which is described in Sanders et al., "Computer-aided segmentation on MRI for prostate radiotherapy, part II: Comparing human and computer observer populations and the influence of annotator variability on algorithm variability," *Radiotherapy and Oncology,* 169 (2022).

Process 900 may include additional implementations, such as any single implementation or any combination of implementations described below and/or in connection with one or more other processes described elsewhere herein.

Although FIG. 9 shows example blocks of process 900, in some implementations, process 900 may include additional blocks, fewer blocks, different blocks, or differently arranged blocks than those depicted in FIG. 9. Additionally, or alternatively, two or more of the blocks of process 900 may be performed in parallel.

### IV. BOUNDING BOX

Embodiments may include a method of detecting for identifying an object in an MR image of a subject, as recited in the appended claims. The method includes receiving an input data structure corresponding to a first MR image, where the first MR image includes a set of image units. Each image unit includes one or more image unit values pertaining to a region of the subject. For each image unit, the input data structure may include a set of properties. The set of properties may include coordinates of the image unit and the image unit value.

The method may include inputting the input data structure into a model. The model may be trained by receiving a plurality of first data structures. Each first data structure may correspond to an MR image. Each first data structure includes values for the same set of properties as the input data structure. The model may be trained by storing a plurality of first training sample. Each first training sample may include one of the first plurality of first data structures and coordinates of a region encompassing an object of interest. The region may be any ellipse, circle, or polygon described herein. The region may be three-dimensional, and may be any spheroid, sphere, or prism described herein. The object of interest is the same or similar to the object in the MR image of the subject. The model may be trained by optimizing, using the plurality of first training samples, parameters of the model based on outputs of the model matching or not matching the coordinates of the region. Outputs of the model specify a region encompassing the object of interest. For example, an output may be coordinates that identify the shape and location of the region encompassing the object of interest.

The method may include determining, using the model, the coordinates of a region encompassing the object in the first MR image. The region may be a bounding box. The regions may be a size that allows for efficient computation across the entire MR image and a size that encompasses the object without encompassing another object. An MR image may have a bounding box for each object in the MR image.

The method may also include determining, using the region, the location of the object. The object may be any object described herein.

The method may include pre-processing or post-processing steps described herein.

Examples of bounding box methods are described in section I.D.

### V. EXAMPLE MEASUREMENT SYSTEMS

**FIG. 10** illustrates a measurement system 1000 according to an embodiment of the present disclosure. The system as shown includes a biological object 1005, such as a region of an organism (e.g., human), within an imaging device 1010, where an emitter 1008 can send waves to biological object 1005. For example, biological object 1005 can receive magnetic fields and/or radio waves from emitter 1008 to provide a signal of a physical characteristic 1015. Biological object 1005 may include objects treated with a contrast agent or other agent to facilitate imaging. An example of an imaging device can be an MRI machine. Imaging device 1010 may include multiple modules.

Physical characteristic 1015 (e.g., an optical intensity, a voltage, or a current), from the biological object is detected by detector 1020. Detector 1020 can take a measurement at intervals (e.g., periodic intervals) to obtain data points that make up a data signal. In one embodiment, an analog-to-digital converter converts an analog signal from the detector into digital form at a plurality of times. Imaging device 1010 and detector 1020 can form an assay system, e.g., a sequencing system that performs sequencing according to embodiments described herein. A data signal 1025 is sent from detector 1020 to logic system 1030. As an example, data signal 1025 can be used to determine locations of particular objects (e.g., organs, tissues, implanted material) in a biological object. Data signal 1025 can include various measurements made at a same time, e.g., different signals for different areas of biological object 1005, and thus data signal 1025 can correspond to multiple signals. Data signal 1025 may be stored in a local memory 1035, an external memory 1040, or a storage device 1045.

Logic system 1030 may be, or may include, a computer system, ASIC, microprocessor, graphics processing unit (GPU), etc. It may also include or be coupled with a display (e.g., monitor, LED display, etc.) and a user input device (e.g., mouse, keyboard, buttons, etc.). Logic system 1030 and the other components may be part of a stand-alone or network connected computer system, or they may be directly attached to or incorporated in a device (e.g., an imaging system) that includes detector 1020 and/or imaging device 1010. Logic system 1030 may also include software that executes in a processor 1050. Logic system 1030 may include a computer readable medium storing instructions for controlling measurement system 1000 to perform any of the methods described herein. For example, logic system 1030 can provide commands to a system that includes imaging device 1010 such that magnetic emission or other physical operations are performed.

Measurement system 1000 may also include a treatment device 1060, which can provide a treatment to the subject. Treatment device 1060 can determine a treatment and/or be used to perform a treatment. Examples of such treatment can include surgery, radiation therapy, chemotherapy, immunotherapy, targeted therapy, hormone therapy, stem cell transplant, and implantation of radioactive seeds. Logic system 1030 may be connected to treatment device 1060, e.g., to provide results of a method described herein. The treatment device may receive inputs from other devices, such as an imaging device and user inputs (e.g., to control the treatment, such as controls over a robotic system).

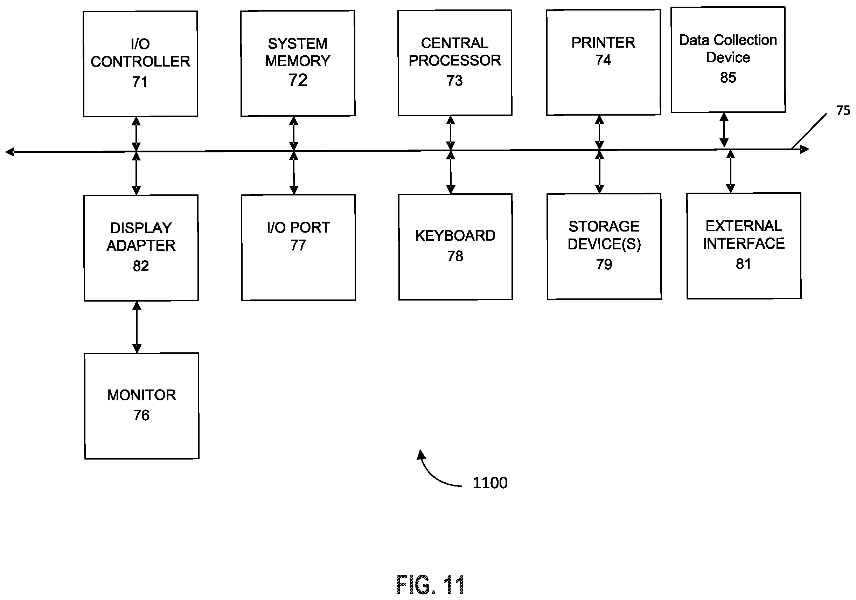

Any of the computer systems mentioned herein may utilize any suitable number of subsystems. Examples of such subsystems are shown in **FIG. 11** in computer system 1100. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in **FIG. 11** are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, Lightning). For example, I/O port 77 or external interface 81 (e.g. Ethernet, Wi-Fi, etc.) can be used to connect computer system 1500 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multicore processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present disclosure using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive, or an optical medium such as a compact disk (CD) or DVD (digital versatile disk) or Blu-ray disk, flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order that is logically possible. Additionally, portions of these steps may be used with portions of other steps from other methods. Also, all or portions of a step may be optional. Additionally, any of the steps of any of the methods can be performed with modules, units, circuits, or other means of a system for performing these steps.

The above description of example embodiments of the present disclosure has been presented for the purposes of illustration and description and are set forth so as to provide those of ordinary skill in the art with a complete disclosure and description of how to make and use embodiments of the present disclosure. It is not intended to be exhaustive or to limit the disclosure to the precise form described nor are they intended to represent that the experiments are all or the only experiments performed. Although the disclosure has been described in some detail by way of illustration and example for purposes of clarity of understanding, it is readily apparent to those of ordinary skill in the art in light of the teachings of this disclosure that certain changes and modifications may be made thereto.

Accordingly, the preceding merely illustrates the principles of the invention. All examples and conditional language recited herein are principally intended to aid the reader in understanding the principles of the disclosure being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein.

A recitation of "a", "an", or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

The claims may be drafted to exclude any element which may be optional. As such, this statement is intended to serve as antecedent basis for use of such exclusive terminology as "solely", "only", and the like in connection with the recitation of claim elements, or the use of a "negative" limitation.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within embodiments of the present disclosure. The upper and lower limits of these smaller ranges may independently be included or excluded in the range, and each range where either, neither, or both limits are included in the smaller ranges is also encompassed within the present disclosure, subject to any specifically excluded limit in the stated range. Where the stated range includes one or both of the limits, ranges excluding either or both of those included limits are also included in the present disclosure.

## Claims

1. A method for identifying an object in a magnetic resonance (MR) image of a subject, the method comprising:
(a) receiving an MR image that includes a set of image units, each comprising one or more image unit values pertaining to a region of the subject, wherein the image unit values form a first distribution;
(b) determining a variation measure of the image unit values in the first distribution;
(c) determining a centroid measure of the image unit values in the first distribution;
(d) identifying a first set of image units having an image unit value that exceeds a threshold corresponding to the variation measure;
(e) discarding the image unit values of the first set of image units;
(f) generating a second distribution centered at the centroid measure;
(g) randomly sampling the second distribution to obtain new image unit values for the first set of image units;
(h) adding the new image unit values to the first set of image units to obtain a new MR image; and
(i) processing the new MR image to identify a location of one or more objects within the subject;
**characterized in that** the MR image includes one or more additional sets of image units, and the set of image units and the one or more additional sets of image units are non-overlapping, and the method further comprises:
repeating steps (b) to (g) for each of the one or more additional sets of image units, and adding the respective new image unit values for each of the one or more additional sets of image units to obtain the new MR images.

2. The method of claim 1, wherein the set of image units is a set of pixels or a set of voxels.

3. The method of any one of claims 1 to 2, wherein determining the variation measure of the image unit values in the first distribution comprises determining the standard deviation of the image unit values in the first distribution.

4. The method of any one of claims 1 to 3, wherein determining the centroid measure of the image unit values in the first distribution comprises determining a mean or a median of the image unit values in the first distribution.

5. The method of any one of claims 1 to 4, wherein the second distribution is a random distribution.

6. The method of any one of claims 1 to 5, wherein the second distribution does not exceed the threshold.

7. The method of any one of claims 1 to 6, wherein the one or more objects comprise one or more radioactive seeds.

8. The method of claim 7, further comprising calculating radiation values for a subset of the set of image units.

9. The method of any one of claims to 8, further comprising:
implanting additional radioactive seeds into the subject.

10. The method of any one of claims 1 to 9, wherein the one or more objects comprise an organ.

11. The method of claim 10, wherein the organ is a prostate, a rectum, a seminal vesicle, an external urinary sphincter, or a bladder.

12. The method of any one of claims 1 to 11, wherein processing the new MR image occurs after radiosurgery on the subject.

13. The method of any one of claims 1 to 12, wherein processing the new MR image comprises determining coordinates of a bounding box around the one or more objects.

## Patentansprüche

1. Verfahren zum Identifizieren eines Objekts in einem Magnetresonanzbild (MR-Bild) eines Probanden, das Verfahren umfassend:
(a) Empfangen eines MR-Bildes, das einen Satz von Bildeinheiten einschließt, die jeweils einen oder mehrere Bildeinheitswerte umfassen, die sich auf einen Bereich des Probanden beziehen, wobei die Bildeinheitswerte eine erste Verteilung bilden;
(b) Bestimmen eines Variationsmaßes der Bildeinheitswerte in der ersten Verteilung;
(c) Bestimmen eines Schwerpunktmaßes der Bildeinheitswerte in der ersten Verteilung;
(d) Identifizieren eines ersten Satzes von Bildeinheiten mit einem Bildeinheitswert, der einen dem Variationsmaß entsprechenden Schwellenwert überschreitet;
(e) Verwerfen der Bildeinheitswerte des ersten Satzes von Bildeinheiten;
(f) Erzeugen einer zweiten Verteilung, die um das Schwerpunktmaß zentriert ist;
(g) zufälliges Abtasten der zweiten Verteilung, um neue Bildeinheitswerte für den ersten Satz von Bildeinheiten zu erhalten;
(h) Addieren der neuen Bildeinheitswerte zu dem ersten Satz von Bildeinheiten, um ein neues MR-Bild zu erhalten; und
(i) Verarbeiten des neuen MR-Bildes, um eine Position eines oder mehrerer Objekte innerhalb des Probanden zu identifizieren;
**dadurch gekennzeichnet, dass** das MR-Bild einen oder mehrere zusätzliche Sätze von Bildeinheiten einschließt, und der Satz von Bildeinheiten und der eine oder die mehreren zusätzlichen Sätze von Bildeinheiten nicht überlappend sind, und das Verfahren ferner umfasst:
Wiederholen der Schritte (b) bis (g) für jeden des einen oder der mehreren zusätzlichen Sätze von Bildeinheiten und Addieren der jeweiligen neuen Bildeinheitswerte für jeden des einen oder der mehreren zusätzlichen Sätze von Bildeinheiten, um die neuen MR-Bilder zu erhalten.

2. Verfahren nach Anspruch 1, wobei die Menge von Bildeinheiten eine Menge von Pixeln oder eine Menge von Voxeln ist.

3. Verfahren nach einem der Ansprüche 1 bis 2, wobei das Bestimmen des Variationsmaßes der Bildeinheitswerte in der ersten Verteilung das Bestimmen der Standardabweichung der Bildeinheitswerte in der ersten Verteilung umfasst.

4. Verfahren nach einem der Ansprüche 1 bis 3, wobei das Bestimmen des Schwerpunktmaßes der Bildeinheitswerte in der ersten Verteilung das Bestimmen eines Mittelwerts oder eines Medians der Bildeinheitswerte in der ersten Verteilung umfasst.

5. Verfahren nach einem der Ansprüche 1 bis 4, wobei die zweite Verteilung eine Zufallsverteilung ist.

6. Verfahren nach einem der Ansprüche 1 bis 5, wobei die zweite Verteilung den Schwellenwert nicht überschreitet.

7. Verfahren nach einem der Ansprüche 1 bis 6, wobei das eine oder die mehreren Objekte einen oder mehrere radioaktive Seeds umfassen.

8. Verfahren nach Anspruch 7, ferner umfassend das Berechnen von Strahlungswerten für eine Teilmenge der Menge von Bildeinheiten.

9. Verfahren nach einem der Ansprüche bis 8, ferner umfassend:
Implantieren zusätzlicher radioaktiver Seeds in den Probanden.

10. Verfahren nach einem der Ansprüche 1 bis 9, wobei das eine oder die mehreren Objekte ein Organ umfassen.

11. Verfahren nach Anspruch 10, wobei das Organ eine Prostata, ein Rektum, eine Bläschendrüse, ein äußerer Harnröhrensphinkter oder eine Blase ist.

12. Verfahren nach einem der Ansprüche 1 bis 11, wobei das Verarbeiten des neuen MR-Bildes nach der Radiochirurgie an dem Probanden erfolgt.

13. Verfahren nach einem der Ansprüche 1 bis 12, wobei das Verarbeiten des neuen MR-Bildes das Bestimmen von Koordinaten eines Begrenzungsrahmens um das eine oder die mehreren Objekte umfasst.

## Revendications

1. Procédé permettant d'identifier un objet dans une image par résonance magnétique (RM) d'un sujet, le procédé comprenant :
(a) la réception d'une image RM qui comporte un ensemble d'unités d'image, chacune comprenant une ou plusieurs valeurs d'unités d'image se rapportant à une région du sujet, dans lequel les valeurs d'unités d'image forment une première distribution ;
(b) la détermination d'une mesure de variation des valeurs d'unités d'image dans la première distribution ;
(c) la détermination d'une mesure de centroïde des valeurs d'unités d'image dans la première distribution ;
(d) l'identification d'un premier ensemble d'unités d'image dont la valeur d'unité d'image est supérieure à un seuil correspondant à la mesure de variation ;
(e) le rejet des valeurs d'unités d'image du premier ensemble d'unités d'image ;
(f) la génération d'une seconde distribution centrée sur la mesure de centroïde ;
(g) l'échantillonnage aléatoire de la seconde distribution afin d'obtenir de nouvelles valeurs d'unités d'image pour le premier ensemble d'unités d'image ;
(h) l'ajout des nouvelles valeurs d'unités d'image au premier ensemble d'unités d'image afin d'obtenir une nouvelle image RM ; et
(i) le traitement de la nouvelle image RM afin d'identifier un emplacement d'un ou plusieurs objets à l'intérieur du sujet ;
**caractérisé en ce que** l'image RM comporte un ou plusieurs ensembles supplémentaires d'unités d'image, et l'ensemble d'unités d'image et le ou les ensembles supplémentaires d'unités d'image ne se chevauchent pas, et le procédé comprend en outre :
la répétition des étapes (b) à (g) pour chacun du ou des ensembles supplémentaires d'unités d'image, et l'ajout des nouvelles valeurs d'unités d'image respectives pour chacun du ou des ensembles supplémentaires d'unités d'image afin d'obtenir les nouvelles images RM.

2. Procédé selon la revendication 1, dans lequel l'ensemble d'unités d'image est un ensemble de pixels ou un ensemble de voxels.

3. Procédé selon l'une quelconque des revendications 1 à 2, dans lequel la détermination de la mesure de variation des valeurs d'unités d'image dans la première distribution comprend la détermination de l'écart-type des valeurs d'unités d'image dans la première distribution.

4. Procédé selon l'une quelconque des revendications 1 à 3, dans lequel la détermination de la mesure de centroïde des valeurs d'unités d'image dans la première distribution comprend la détermination d'une moyenne ou d'une médiane des valeurs d'unités d'image dans la première distribution.

5. Procédé selon l'une quelconque des revendications 1 à 4, dans lequel la seconde distribution est une distribution aléatoire.

6. Procédé selon l'une quelconque des revendications 1 à 5, dans lequel la seconde distribution ne dépasse pas le seuil.

7. Procédé selon l'une quelconque des revendications 1 à 6, dans lequel le ou les objets comprennent un ou plusieurs grains radioactifs.

8. Procédé selon la revendication 7, comprenant en outre le calcul de valeurs de radiation pour un sous-ensemble de l'ensemble d'unités d'image.

9. Procédé selon l'une quelconque des revendications 8, comprenant en outre :
l'implantation de grains radioactifs supplémentaires dans le sujet.

10. Procédé selon l'une quelconque des revendications 1 à 9, dans lequel le ou les objets comprennent un organe.

11. Procédé selon la revendication 10, dans lequel l'organe est une prostate, un rectum, une vésicule séminale, un sphincter urinaire externe ou une vessie.

12. Procédé selon l'une quelconque des revendications 1 à 11, dans lequel le traitement de la nouvelle image RM a lieu après une radiochirurgie sur le sujet.

13. Procédé selon l'une quelconque des revendications 1 à 12, dans lequel le traitement de la nouvelle image RM comprend la détermination de coordonnées d'une zone de délimitation autour du ou des objets.
